(19) Europäisches Patentamt
European Patent Office
Office européen des brevets

(11) EP 0 421 935 B1

(12) EUROPEAN PATENT SPECIFICATION

(45) Date of publication and mention of the grant of the patent: **07.01.1999 Bulletin 1999/01**

(51) Int Cl.⁶: **C12N 15/16**, C12N 15/86, A01N 63/00, C07K 7/08, C07K 7/00

(21) Application number: **90810745.1**

(22) Date of filing: **28.09.1990**

(54) **Insect neuropeptides**

Insekt-Neuropeptide

Neuropeptides de l'insecte

(84) Designated Contracting States:
**BE CH DE DK FR GB IT LI NL**

(30) Priority: **02.10.1989 US 416097**

(43) Date of publication of application:
**10.04.1991 Bulletin 1991/15**

(73) Proprietor: **Novartis AG**
**4058 Basel (CH)**

(72) Inventors:
- **Kramer, Steven Jan**
  **Sunnyvale, CA 94087 (US)**
- **Miller, Christine Ann**
  **Campbell, CA 95008 (US)**
- **Schooley, David Allan**
  **Reno, Nevada 89503 (US)**
- **Toschi, Anne Gregurech**
  **Burlingame, CA 94010 (US)**

(56) References cited:
- **MOLECULAR AND CELLULAR ENDOCRINOLOGY vol. 49, no. 2,3, February 1987, SHANNON, IRELAND pages 237 - 248; N.A. GRANGER AND W.P. JANZEN: 'Inhibition of Manduca Sexta corpora allata in vitro by a cerebral allatostatic neuropeptide'**
- **AGRICULTURAL AND BIOLOGICAL CHEMISTRY. 1987, TOKYO JP pages 1067 - 1076; H. KATAOKA ET AL.: 'Isolation and partila characterization of a prothoracicotropic hormone of the silkworm Bombyx mori'**
- **GENERAL AND COMPARATIVE ENDOCRINOLGY vol. 78, no. 1, April 1990, NEW YORK, NY, USA pages 123 - 136; G. BHASKARAN ET AL.: 'Allatoinhibin, a neurohormonal inhibitor of juvenile hormone biosynthesis in Manduca Sexta'**
- **PROCEEDINGS OF THE NATIONAL ACADEMY OF SCIENCES OF USA. vol. 86, August 1989, WASHINGTON US pages 5997 - 6001; A.P. WOODHEAD ET AL.: 'Primary structure of four allatostatins: Neuropeptide inhibitors of juvenile hormone synthesis'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to the isolation and characterization of lepidopteran allatostatin neuropeptide. The isolated allatostatin is a polypeptide which directly or indirectly inhibits juvenile hormone (JH) synthesis in Lepidoptera. Means for recombinant production and utilization of polypeptides having allatostatin activity are also provided.

A method that utilizes the in vitro culture of larval Corpora allata of manduca sexta and an RIA for quantification of JH biosynthesis has been established to investigate the potential humoral regulation of JH synthesis at the level of the gland by Granger et al., Molecular and Cellular Endocrinology, 16, 1-17, (1979).

The isolation of four neuropeptides that inhibit juvenile hormone synthesis by the corpora allata from brains of the virgin female cockroach Diplotera punctata and the sequences thereof have been discribed by Woodhead et al., PNAS, 86, 5997-6001.

### Brief Description of the Invention

Lepidopteran allatostatin (LAS) is extracted from heads of the tobacco hornworm Manduca sexta and then purified using a multi-step procedure involving ion exchange column chromatography, reversed-phase chromatography, and liquid chromatography (semi-preparative, cation-exchange, analytical and microbore). The product is a substantially homogenous polypeptide.

After purification, the LAS polypeptide was analyzed and its amino acid sequence was determined. The native peptide was found to involve a sequence of 15 amino acids and to have a free carboxyl terminus.

Synthetically produced or isolated LAS may be used as insecticides and insect population control agents by directly or indirectly affecting juvenile hormone in the insect. LAS is particularly effective against Lepidopteran species.

The following definitions will apply throughout the specification and claims:

LAS activity or LAS-like activity - the activity of a polypeptide observed in the in vitro JH biosynthesis assay described herein which mimics or evokes substantially the same type of response as native LAS from Manduca sexta in said assay.

Native LAS - a polypeptide which is found in Lepidoptera which is directly or indirectly involved in the regulation of Juvenile hormone synthesis.

LAS Analog (LASA) - a polypeptide which does not have the same aminoacid sequence as native LAS or is not coded by the same DNA sequence but possesses LAS or LAS-like activity.

Associated insect polypeptides - polypeptides occurring naturally in an insect which are other than LAS or possess no LAS or LAS-like activity.

### Detailed Description of the Invention

One aspect of this invention provides for a polypeptide having LAS-like activity which is substantially free from associated insect polypeptides.

The LAS-like activity is further characterized by an ability of the polypeptides according to the invention to inhibit synthesis of juvenile hormone (JH) I, II and III in lepidoptera.

The polypeptides according to the invention may additionally be characterized by having a molecular weight of <2kDa.

Naturally occurring Native LAS as isolated from Manduca sexta has the following amino acid sequence:

```
pGlu-Val-Arg-Phe-Arg-Gln-Cys-Tyr-Phe-Asn-Pro-Ile-Ser-Cys-Phe    (I)
  1   2   3   4   5   6   7   8   9   10  11  12  13  14  15
```

The carboxy terminus (on Phe) of Native LAS is in free form but the amidated form possesses the same level of activity and also forms part of the invention.

It has been found that LAS-Analogs exist or may be synthesized which possess LAS-like activity. These comprise modification (eg deletions and/or substitutions) of the Native LAS amino acid sequence provided that Cys residues at positions 7 and 14 are conserved and are flanked by at least one amino acid and separated by 6 amino acids.

These LASA's may be characterized by the following formula A

$$AA_1\text{-}AA_2\text{-}AA_3\text{-}AA_4\text{-}AA_5\text{-}AA_6\text{-}Cys\text{-}AA_8\text{-}AA_9\text{-}AA_{10}\text{-}AA_{11}\text{-}AA_{12}\text{-}AA_{13}\text{-}Cys\text{-}AA_{15} \quad (A)$$

wherein each of $A_6$ to $A_{15}$ is independently an amino acid selected from Val, Ile, Leu, Met, Phe, Pro, Trp, Asn, Gln,

Gly, Ser, Thr, Arg, His, Lys, Asp or Glu; and
each of $AA_1$ to $AA_5$ is independently an amino acid selected from the meanings given for $A_6$ to $A_{15}$ or if it is the terminal group may be hydrogen and by possessing LAS-like activity.

A particular sub-group (compounds $A_1$) within the compounds of formula A comprises those compounds wherein each of $AA_6$ to $AA_{15}$ is independently an amino acid selected from Gln, Tyr, Phe, Asn, Pro, Ile, Ser, Phe or Ala; and each of $AA_1$ to $AA_5$ is independently an amino acid selected from Glu, Val, Arg, Phe, Ala or Lys or if it is the terminal group may be hydrogen.

A further subgroup (compounds $A_2$) within the compounds of formula A comprises those compounds wherein $AA_1$ is pGlu, $AA_2$ is Val, $AA_6$ is Gln, $AA_9$ is Phe, $AA_{10}$ is Asn, $AA_{12}$ is Ile, $AA_{13}$ is Ser, $AA_{15}$ is Phe and

a) $AA_3$ and $AA_5$ are both Arg or Lys, $AA_4$ is Phe or Ala, $AA_8$ is Tyr or Phe and $AA_{11}$ is Pro or Ala; or
b) $AA_3$ and $AA_5$ are both Arg or Lys, $AA_4$ is Phe, $AA_8$ is Tyr and $AA_{11}$ is Pro; or
c) $AA_3$ and $AA_5$ are both Arg or Lys, $AA_4$ is Phe or Ala, $AA_8$ is Tyr and $AA_{11}$ is Pro; or
d) $AA_3$ and $AA_5$ are both Arg, $AA_4$ is Phe, $AA_8$ is Tyr or Phe and $AA_{11}$ is Pro; or
e) $AA_3$ and $AA_5$ are both Arg, $AA_4$ is Phe, $AA_8$ is Tyr and $AA_{11}$ is Pro or Ala.

Yet a further subgroup (compounds $A_3$) within the compounds of formula A comprises those compounds wherein $AA_6$ to $AA_{15}$ comprise the sequence Gln-Cys-Tyr-Phe-Asn-Pro-Tle-Ser Cys-Phe and $AA_1$ to $AA_5$ comprise a sequence selected from pGlu; Arg; Phe-Arg; Arg-Phe-Arg; Val-Arg-Phe-Arg; pGlu-Val-Arg-Phe-Arg; Val-Arg-Arg; or pGlu-Arg-Phe-Arg.

Examples of particular compounds of formula A are as follows (AS=Native LAS):

[amino acids 2-15 of] AS; [3-15] AS; [4-15] AS; [5-15] AS; pGlu [6-15] AS; pGlu-Val-Arg-[5-15] AS; pGlu [3-15] AS; [1-7] AS-Phe-[9-15] AS; [1-10] AS-Ala-[2-15] AS; pGlu-Val-Lys-Phe-Lys[6-15] AS; pGlu-Val-Arg-Ala-[5-15] AS.

In all LAS and LASA's the Cys residues at positions 7 and 14 are preferably linked by a disulphide bridge to provide the tertiary structure for the desired activity. For convenience this bridge is not shown.

As in the case of Native LAS the compounds of formula A, $A_1$, $A_2$ and $A_3$ may have the carboxy terminus in free form or in amidated form.

As will be noted in the formulae above Glutamine (Glu) when in terminal position may be, and preferably is, present in cyclic form as pyroglutamine (pGlu).

The biological activity of native LAS and LASA's is tested in an in vitro JH-biosynthesis assay using newly molted 5th stadium Manduca sexta larval CC/CA complex.

This invention also provides the use of LAS and LASA as insecticides or as agents to enhance the activity of other insecticides. A composition for such purposes comprises an effective amount of LAS or LASA combined with an inert carrier which does not adversely affect the active peptide ingredient, but which preferably facilitates contact uptake or absorption of the LAS or LASA, such as DMSO and water. This composition is applied to the insect or its environment. Typical dosage would range from 0.5-50 kg/hectare, preferably 2-20 kg/hectare, with applications repeated as required. LAS and LASA are particularly useful when produced by expression in a recombinant hybrid virus to enhance the insecticidal activity of the virus.

Also provided by the invention are DNA sequences encoding LAS and polypeptides having LAS activity, and especially such DNA when combined with heterologous DNA to form systems for the expression, production and/or delivery of polypeptides which have LAS-like activity (including LAS itself). Such DNA sequences may be routinely constructed from the knowledge of the genetic code and codon bias for the insect species involved with the use of conventional DNA synthesizers and ligation of preformed oligonucleotides. The term "heterologous DNA" refers to DNA not associated with the DNA for the production of LAS polypeptides in nature (or a structural gene for an analog thereof). Heterologous DNA typically involves promoter, signal or operator sequences but may simply involve plasmid or vector DNA not operatively involved in expression.

A variety of nucleotide sequences may be constructed to encode the amino acid sequences of LAS or LASA and their signal and stop sequences. Hence different codons encoding the same amino acid in such amino acid sequence may be substituted as allowed by the genetic code. All of such codon or nucleotide changes while retaining a sequence coding for the amino acids of LAS or LASA will nevertheless result in a nucleotide or DNA sequence which are highly homologous.

As discussed in detail above and as contemplated herein, various changes may be made in the amino acid sequence of LAS as depicted by Formula I while still providing a polypeptide having the type of biological activity of LAS. Such modified polypeptides are herein referred to as LAS-analogs (LASA) and evoke substantially the same type of anti JH response in the bioassays described herein as the native mature LAS. They include deletions of one or more amino acids such as those at or near the N-terminus, substitutions of one or more amino acids for another involving for example exchange of homologous natural amino acids or substitutions for amino acids which are not essential for

LAS activity and additions or extensions by one or more amino acids of the native amino acid sequence such as extensions at the N-terminus. DNA sequences having a coding or sense strain encoding such LASA polypeptides may be constructed in conventional manner e.g. by linking of synthetic oligonucleotides. Such DNA sequences encoding a LASA polypeptide are preferably and particularly those which will also hybridize under stringent hybridization conditions to a DNA segment having or incorporated in one of the two strands thereof the same nucleotide sequence encoding the native mature LAS depicted. Stringent hybridization conditions as contemplated herein are those in which hybridization is effected in a standard manner at 68°C in 6.0 X saline citrate buffer followed by merely rinsing at 37°C at reduced buffer concentration (which will not affect the hybridizations which have taken place). The DNA to be used for hybridization may be prepared in a conventional manner and may be tagged to form an identifiable hybridization probe by procedures well known in the art. Such a probe may also be used to probe suitable and conventionally prepared clone banks to locate and isolate alleles (both nucleotide and amino acid alleles) of the LAS of M. sexta or to locate and isolate genes coding for LAS in other or related insect species. Selection of particular codons for particular amino acids is preferably in accord with natural preferences for the cell in which expression is desired.

A particular method for use of LAS or LASA as an insecticide is using an insect-specific virus, especially baculovirus as a vector. For example, a gene encoding LAS or LASA is inserted into a baculovirus DNA, and is under the control of a baculovirus promoter. After an insect ingests the recombinant hybrid baculovirus DNA, the virus DNA multiplies inside the insect, and LAS or LASA is expressed (produced) in a sufficient amount as to enhance the insecticidal effect of the virus DNA. Such recombinantly modified baculovirus DNA may also be used as vectors to transform or transfect cells, particularly insect cells, to provide systems for the production of LAS or LASA.

A number of baculovirus are known in the art, which would be suitable for use as vectors, including Autographa californica Nuclear Polyhedrosis Virus (AcMNPV) and Bombyx mori Nuclear Polyhedrosis Virus (BmNPV). The codon preferences for the particular virus chosen can be determined by comparing codon usage in known baculovirus sequences. A structural gene for LAS or LASA is then synthesized followed by a stop codon, preferably utilizing these codon preferences. The structural gene is preferably ligated to a signal or secretion sequence of the type to provide for the transport of expressed protein through the cell membrane, such sequence being processable upon secretion to cleave the sequence from the proform to provide the mature product. Many signal sequences are known in the art including the signal sequence associated with larval cuticle protein 2 of the fruit fly Drosophila melanogaster, reported by Snyder et al., 1982 Cell v29 1027-1040. This sequence can be used in its native form, or preferably, can be altered so that its codons conform to those preferred by the baculovirus.

The signal sequence and structural gene are placed downstream from a desired baculovirus promoter. Examples of suitable promoters include the NPV polyhedron gene promoter. One method of inserting the LAS construct is by using a transfer or shuttle vector containing a LAS or LASA gene sequence flanked on at least one and preferably on both sides by a region of baculovirus DNA. A double crossover or cotransfection occurs upon combining the virus DNA and shuttle vector under cotransfection conditions. The result is a recombinant hybrid baculovirus containing an expressible LAS or LASA gene.

In order to construct a desired intermediate plasmid, successive DNA-fragments encoding portions of LAS or LASA and having overhanging nucleotides to facilitate ligation are ligated with various fragments obtained from known plasmids such as pTZ19R and pTZ18R (Pharmacia Inc.) employing appropriate endonucleases in conventional manner. Such a plasmid can be used to transform bacteria to obtain copies and confirm structure.

The LAS gene including the secretion (signal) sequence therefor as available in the intermediate plasmid is then excised therefrom and placed under transcriptional control of polyhedron gene sequences obtained from baculovirus DNA. A shuttle vector containing the resulting recombinant gene is contacted with baculovirus DNA to effect a recombination or crossover resulting in incorporation of recombinant gene into the baculovirus genome. The resulting modified or hybrid baculovirus DNA is recovered and multiplied and may be used as expression vectors to infect (transform or transfect) insect cells, such as Autographa californica cells, to produce LAS and LASA. The basic method for preparation of such modified viruses is known and described for example in U.S. patent 4,745,051 and in Manual of Methods for Baculovirus Vectors and Insect Cell Culture Procedures, Summers & Smith, Texas A&M University, 1988. The ability of the LAS-like polypeptides to be expressed in active form in such systems and/or enhance insecticidal activity is judged unexpected.

Any of the polyhedron gene containing Baculoviruses (nuclear polyhedrosis viruses or NPV) may be used in preparing such vectors and insecticides, including Autographa Californica NPV as described in detail in U.S. patent 4,745,051. Another system based on Bombyx mori NPV is also of interest and basic information thereon is available, see, for example, published European Patent Application 0175852, and may be employed to demonstrate the use of NPV's for expression of LAS and the LASA polypeptides and enhancement of insecticidal activity of the NPV therewith. Other suitable baculoviruses are Heliothis zea e.g. as described in WO 88/07087 or celery looper virus as described in USP 4,911,913. Other baculovirus types and preparation techniques are described in EP 0155476, EP 0228036, WO 88/07082, WO 89/01518, NL 8800198, EP 340359.

In the practice of the present invention the preferred baculovirus system is Autographa californica.

By way of illustration the following scheme outlines the construction of a recombinant plasmid vector pVLAS containing the pre-allatostatin gene. This proceeds starting from the commercially available plasmids pTZ19R and pTZ18R (Pharmacia Inc.) through intermediate plasmids pATI, pAT2, pATC and pASI to allow isolation of the pre-allatostatin gene which is then cloned into the baculovirus transfer vector pVL1392 (Invitrogen Corp.). pVL1392 is a multiple cloning site (MCS) containing derivative of the baculovirus transfer vector pVL941, a plasmid in which the ATG initiation codon for the polyhedrin gene was changed to ATT by site directed mutogenesis [Luckow & Summers; Virology. 170, 31-39 (1989)]. Approximate coordinates (in base pairs) are indicated on the pVL1392 map.

The following synthetic oligonucleotides were used in the construction of the pre-allatcstatin gene.

SIGC: 5'-AATTCAATATGTTCAAGTTCGTTATGATCCTGGCTGTGGTTGGCGTCGCTACTGCA-3'

SIGNC: 5'-GTAGCGACGCCAACCACAGCCAGGATCATAACGAACTTGAACATATTG-3'

ASC: 5'-GAGGTGCGTTTCCGTCAATGCTACTTCAACCCCATCAGCTGCTTCTAAT-3'

ASNC: 5'-CTAGATTAGAAGCAGCTGATGGGGTTGAAGTAGCATTGACGGAAACGCACCTCTGCA-3'

ATC: 5'-GGATTCAAGAACGTTGAGATGATGACCGCTCGTGGATTCGGTTAAT-3'

ATNC: 5'-CTAGATTAACCGAATCCACGAGCGGTCATCATCTCAACGTTCTTGAATCCTGCA-3'

SIGC and SIGNC correspond to the coding and non-coding strands, respectively, of the Drosophila cuticle protein gene signal peptide. ASC and ASNC correspond to coding and noncoding strands, respectively, of mature allatostatin. ATC and ATNC correspond to coding and noncoding strands, respectively, of mature allatotropin.

The techniques employed are eg as described in the art as cited herein or as described hereinafter in the examples.

Construction of pVLAS

5

1. Cut pTZ19R with EcoRI and PstI
2. Ligate with synthetic Drosophila cuticle protein signal peptide gene

3. Cut pTZ18R with PstI and XbaI
4. Ligate with synthetic allatotropin gene

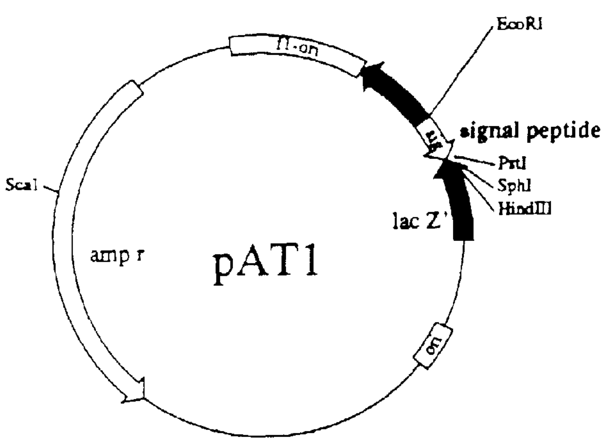


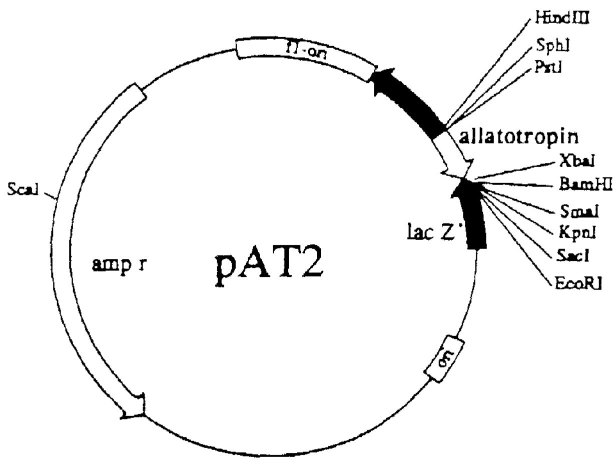


5. Cut pAT1 with PstI and ScaI
6. Isolate the small PstI-ScaI fragment

7. Cut pAT2 with PstI and SmaI
8. Isolate the large PstI-ScaI fragment

9. Ligate the isolated fragments

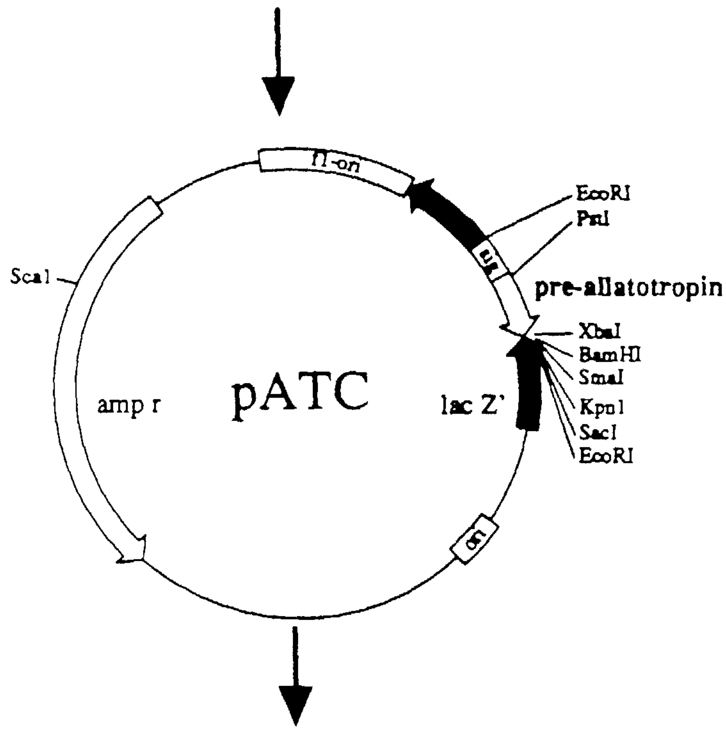


10. Cut pATC with PstI and XbaI
11. Isolate the large PstI-XbaI fragment
12. Ligate with synthetic allatostatin gene

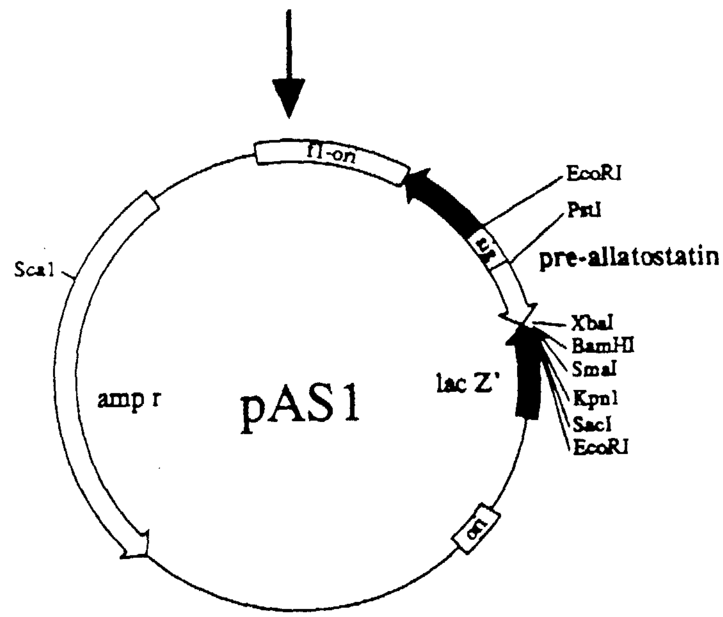


13. Cut pAS1 with EcoRI and SmaI
14. Isolate the small fragment containing the pre-allatostatin gene

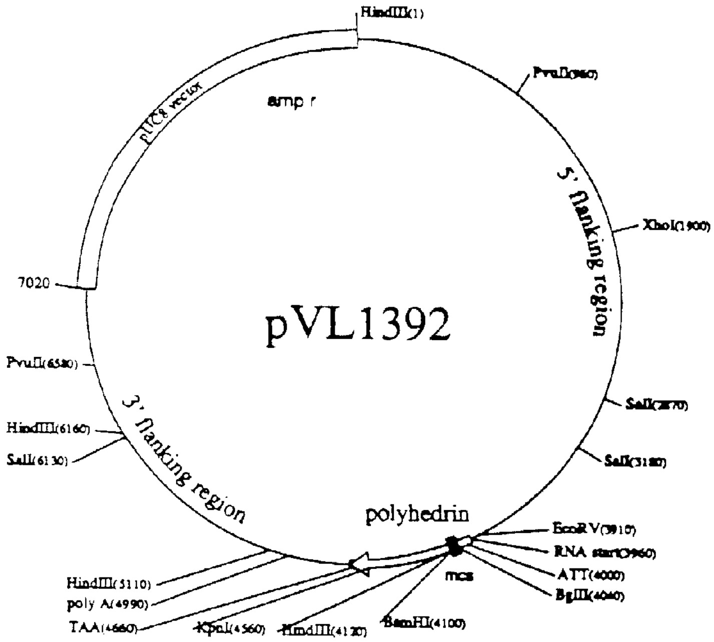


Multiple cloning site (MCS) — -------TATAAATATTCCGGATTATTCATACCGTCCCACCATCGGGCGC

BglII NotI EcoRI BamHI
GATCAGATCTGCAGCGGCCGCTCCAGAATTCTAGAAGGTACCCGGGATCCT
PstI XbaI SmaI

TCCT----------

15. Cut pVL1392 with EcoRI and SmaI
16. Ligate with the small fragment isolated in step 14 to form pVLAS.

pVLAS: pre-allatostatin gene and adjacent sequences

-------TATAAATATTCCGGATTATTCATACCGTCCCACCATCGGGCGCG

BglII NotI EcoRI
GATCAGATCTGCAGCGGCCGCTCCAGAATTCAAT**ATGTTCAAGTTCGTT**
PstI

**ATGATCCTGGCTGTGGTTGGCGTCGCTACTGCAGAGGTG**
PstI

**CGTTTCCGTCAATGCTACTTCAACCCCATCAGCTGCTTC**T

BamHI BamHI
AATCTAGAGGATCCCCGGGATCCTT-----
XbaI SmaI

The shuttle vector containing an LAS or LASA gene is then exposed to the viral DNA of AcMNPV to transfect the viral DNA with the LAS or LASA-containing AcMNPV sequences.

The LAS or LASA polypeptide may be produced biotechnologically from the LAS or LASA gene-containing modified baculoviruses of the invention in essentially a known manner. Insect cells are cultured (grown) to about the final volume desired for production, then infected with the modified baculovirus DNA of the invention and the resulting infected (transformed or transfected) cells maintained to allow expression (production) of the LAS or LASA. Such processes

are run on a batch basis in view of the destructive effect of the virus on the cells.

The modified baculovirus of the invention may be themselves used as insecticides in the general manner in which the unmodified native viruses may be used, i.e. by application of an effective amount of the locus of insects susceptible to the virus. The modified viruses as produced above have been found viable or competent for such purposes by containing native DNA sequences for reproduction, lethal infestation and expression of the LAS or LASA polypeptide. The modified viruses may be advantageously also combined with and otherwise coapplied with the unmodified, native viruses or with other viral proteins which enhance their effect (cf EP 336,341). These viruses may be further modified eg. to delete the so-called EGT gene (cf O'Reilly & Miller; Science, v. 245 pp. 1110-2) to prevent blocking of insect molt (L. K. Miller, Proc. Vth International Colloquium on Invertebrate Pathology and Microbial Control, Adelaide, August 1990, p.446). Insecticidal compositions containing such modified or native baculovirus in an insecticidally effective amount will typically comprise inert carriers such as clay, lactose and proteinaceous materials such as defatted soybean powder to assist in application, particularly those carriers such as the proteinaceous materials which are feeding attractants and which may enhance stability of the contained baculoviruses. Such compositions are desirably stored at presentation of the modified and unmodified viruses. Such suitable compositions are further described for example in U.S.P. 4,716,039. The following examples illustrate the invention without in any way restricting the scope thereof. Alternative analogous methods for carrying out the procedures described herein are described in EP 359,714 (US Serno 07/245,429), the contents of which in this respect is incorporated herein by reference.

**EXAMPLE A**

The biological activity of each of the LAS and LASA preparations made according to the following examples is assayed according to a procedure essentially as described (except for media employed) by Pratt and Tobe, 1974 Life Sci vl4:575-586, which is incorporated by reference. This procedure measures the rate of biosynthesis of Juvenile Hormone (JH) by the corpora allata which have been surgically removed from newly molted 5 stadium Manduca sexta larvae.

One corpus cardiacum-corpus allatum complex is maintained in 100 µl of TC-199 medium (components of which are shown below) and Hank's salts, and is shaken using a mechanical shaker. The sample to be tested for LAS-like activity is dried and dissolved in TC-199 medium. Then an equal volume of TC-199 medium containing [$^{14}$C] methionine is added, and allowed to incubate for 4 hours.

[$^{141}$C]JH is extracted from the medium using iso-octane and the extract is assayed by liquid scintillation counting. The rate of biosynthesis by glands exposed to the sample is compared to the rate observed for control glands. The presence of LAS or LASA causes a decrease in the rate of biosynthesis.

**EXAMPLE B**

The assay for detection/evaluation of the insect control (insecticidal) activity of LAS and LASA is based on contact and insect penetration of the LAS and LASA. DMSO is employed as a carrier to facilitate such uptake. Accordingly, the LAS or LASA to be evaluated was dissolved in about 30% aqueous DMSO and applied at various concentrations (1 mg/ml and ten serial dilutions thereof) to a test group of twenty stage-synchronized lepidopteran insects (Manduca sexta), at about the beginning of the third instar stage at about 27°C. The insects are evaluated for retarded growth and survival over the remaining molting stages and compared with a control group treated only with the DMSO solution.

| Composition of TC-199 Medium | |
|---|---|
| Component | mg/L |
| L-Histidine HCl.$H_2O$ | 21.88 |
| L-Hydroxyproline | 10.00 |
| D,L-Isoleucine | 40.00 |
| D,L-Leucine | 120.00 |
| L-Lysine HCl | 70.00 |
| D,L-Methionine | 30.00 |
| D,L-Phenylalanine | 50.00 |
| L-Proline | 40.00 |
| D,L-Serine | 50.00 |
| D,L-Threonine | 60.00 |
| D,L-Tryptophan | 20.00 |

(continued)

| Composition of TC-199 Medium | |
|---|---|
| Component | mg/L |
| L-Tyrosine | 40.00 |
| D,L-Valine | 50.00 |
| Ascorbic Acid | 0.05 |
| Alpha tocopherol phosphate (disodium salt) | 0.01 |
| d-Biotin | 0.01 |
| Calciferol | 0.10 |
| D-Ca pantothenate | 0.01 |
| Choline chloride | 0.50 |
| Folic Acid | 0.01 |
| i-Inositol | 0.05 |
| Menadione | 0.01 |
| Niacin | 0.025 |
| Niacinamide | 0.025 |
| Para-aminobenzoic acid | 0.05 |
| Pyridoxal HCl | 0.025 |
| Pyridoxine HCl | 0.025 |
| Riboflavin | 0.01 |
| Thiamine HCl | 0.01 |
| Vitamin A (acetate) | 0.14 |

**EXAMPLE 1**

Extraction and Preliminary Purification

Pharate adult M. sexta are beheaded 24-48 hours before adult eclosion, and the heads frozen. A posterior section of the frozen heads containing the brain and the corpora cardiaca/corpora allata complex is punched out with a 5 mm diameter cork borer and stored at -80°C until extraction.

Thirty thousand trimmed heads of M. sexta (fresh weight approximately 1460 g) are homogenized in total volume 1.5 l cold acetone and filtered. Residues are extracted with 1.4 l of 1 M HOAc/20 mM HCl (containing 0.1 mM phenylmethylsulfonyl fluoride and 0.01 mM pepstatin A, prepared freshly) and centrifuged at 10000X g for 30 min. After re-extraction of the pellet with total volume of 0.9 l of the same solution, and recentrifugation, the combined supernatants are applied to a SP-Sephadex C-25 column (25 x 700 mm, 300 ml bed volume) equilibrated with 1 M HOAc. The column is eluted with 1 l each of 0.1, 0.2 and 0.4 M $NH_4OAc$ (pH 7.0). The $NH_4OAc$ fractions, which have LAS activity, are applied directly to 10 g of reversed-phase Vydac $C_4$ packing material (20-30 µm, contained in a 75 ml polypropylene syringe barrel with polyethylene frit) equilibrated with 0.1% trifluoroacetic acid (TFA). The cartridge is eluted with 100 ml of 60% $CH_3CH$ in 0.1% TFA. LAS is recovered in the 60% $CH_3CH$/0.1% TFA fraction.

**EXAMPLE 2**

Purification TSK SP-5PW prep LC 1 Vydac $C_4$ Semipreparative Liquid Chromatography (LC)

This and subsequent purifications by LC are performed with a Perkin-Elmer Model 410 Bio pump, a Rheodyne loop injector, and a Kratos Model 783 variable wavelength detector, set at 220 nm. The active fraction from Example 1 is diluted 1:1 with water and pumped on to a TSK SP-SPW 2.15 X 5 cm column previously equilibrated with water. The column is eluted with a 90 minute linear gradient of 0.1 M NaCl in 0.1 M MES buffer pH 5.5 at a flow rate of 8 ml/min. 12 ml fractions are collected. LAS activity is found in fractions which elute from 31-37 minutes.

These are combined and diluted 1:1 with 0.1% TFA and pumped on to a Vydac $C_4$ semipreparative column (10 x 250 mm) previously equilibrated with 0.5% HFBA. The column is eluted with a 60 min. linear gradient of 20-26% 1-PrOH in 0.5% HFBA at a flow rate of 5 ml/min; 10 ml fractions are collected. LAS activity is smeared in fractions which elute from 48-60 minutes.

Active fractions from previous runs are combined and diluted 1:2 with 0.1% TFA and pumped on a Vydac $C_4$

semiprep column previously equilibrated with 0.1% TFA. The column is eluted with a 60 min linear gradient of 20-35% Acetonitrile in 0.1% TFA at a flow rate of 5 ml/min; 10 ml fractions are collected. LAS activity is found in fractions which elute at 52-54 minutes.

TSK SP-SPW LC2

The active fractions from the previous step are diluted 1:1 with water and pumped on to a TSK SP 5PW column (7.5 x 0.75 mm), equilibrated with 20 mM MES (pH 5.5) containing 10% $CH_3CN$. The column is eluted with a gradient at a flow rate of 1 ml/min; 40 min of 0.2-0.5 M LiCl in the same buffer. Two ml fractions are collected. LAS is recovered in the 26-28 min fractions.

Vydac $C_4$ Microbore LC

The active fraction from the previous step are applied to a Vydac $C_4$ microbore column (2.1 x 250 mm). The column is equilibrated with 10% 1-PrOH in 0.1% TFA. After elution with a 60 min linear gradient of 10-25% 1-PrOH/0.1% TFA at a flow rate of 0.2 ml/min pure LAS is recovered in a single peak at 41-42 min.

**EXAMPLE 3**

Sequence Analysis

Purified LAS from Example 2 is sequenced using an Applied Biosystems Model 477A pulsed liquid-phase protein sequencer. Released phenylthiohydantoin (PTH) amino acids are analyzed using an on-line analyzer (Applied Biosystems, Model 120A).

**EXAMPLE 4**

Amino Acid Analyses

Purified LAS is hydrolyzed in vapor from 6 M HCl/1% phenol (110°C for hr). Hydrolysates are analyzed after conversion to phenylthiocarbamoyl amino acid derivatives by reversed-phase LC using an Ultrasphere ODS column (4.6 x 150 mm).

The starting buffer (D) is 0.05 M sodium acetate containing 1.15 mM triethylamine and 2% methanol, adjusted to pH 6 with phosphoric acid. The eluting buffers are identical except for the organic solvent; B contains 50% methanol and C contains 50% $CH_3CN$. The column washing solvent (A) is 70% $CH_3CN$ is $H_2O$. Buffers are delivered by a Perkin-Elmer series 4 pump using the following program.

| Time | Flow | A | B | C | D |
|---|---|---|---|---|---|
| 0 min | 1.0 ml/min | 0 | 0 | 0 | 100% |
| 21 min | 1.0 ml/min | 0 | 30% | 0 | 70% |
| 30 min | 1.0 ml/min | 0 | 0 | 35% | 65% |
| 45 min | 1.0 ml/min | 0 | 0 | 49% | 51% |
| 55 min* | 1.5 ml/min | 100% | 0 | 0 | 0 |
| 71 min* | 1.0 ml/min | 0 | 0 | 0 | 100% |
| 79 min* | 1.0 ml/min | 0 | 0 | 0 | 100% |

*Not gradient, isocratic segments

Analysis of the isolated peptide gives the following results.

| Amino Acid Composition LAS | | |
|---|---|---|
| | found rel. to Val | integral assignment |
| Asx | 1.4 | (1) |
| Glx | 3.0 | (3) |
| Ser | 2.1 | (2) |
| His | 0.4 | (0) |

(continued)

| Amino Acid Composition LAS | | |
|---|---|---|
| | found rel. to Val | integral assignment |
| Gly | 2.0 | (2) |
| Thr | 0.7 | (1) |
| Ala | 0.9 | (1) |
| Arg | 2.2 | (2) |
| Tyr | 1.2 | (1) |
| Val | 1.0 | (1) |
| Met | 0.3 | (0) |
| Ile | 1.1 | (1) |
| Phe | 2.5 | (3) |
| Leu | 0.5 | (1) |
| Lys | 0.5 | (1) |
| Pro | 1.1 | (1) |
| Cys | - | (2) |
| | | 23 |
| Asx = Asn/Asp; Glx = Gln/Glu | | |

This amino acid composition exceeds the composition of the sequenced peptide (15 amino acids). To solve this discrepancy LAS was reduced and the Cys residues were carboxymethylated with tritiated iodoacetic acid. Amino acid analysis of this RCM peptide was consistent with a 15 amino acid synthetic peptide.

The nature of the carboxyl terminus of LAS was established by comparison of reversed-phase liquid chromatographic (RPLC) retention times of the reduced and carboxylated forms of native LAS and synthetic C-terminal acid and amide LAS peptides. Native RCM-LAS coelutes with the synthetic RCM acid form when coinjected. Thus the complete structure of LAS is pGlu-Val-Arg-Phe-Arg-Gln-Cys-Tyr-Phe-Asn-Pro-Ile-Ser-Cys-Phe-OH.

The additional amino acids found an analysis of native LAS may be contributed by coeluting additional peptides present in lesser amounts.

LAS activity in fractions from each purification step of Example 2 is detected using the in vitro assay described in Example A.

## EXAMPLE 5

Synthesis of peptides

All LAS and LASA are synthesized by the solid phase method, using a Biosearch Model 9600 peptide synthesizer and a tert-butoxycarbonyl (Boc) chemistry protocol. For carboxyl-terminal amides, para-methylbenzhydrylamine (PMBHA) resin hydrochloride is used as the source of the amide nitrogen and as the solid support. For the corresponding carboxyl-terminal acid, Boc-Phe-O-resin is used to initiate the peptide sequence. The amino acid side chains are protected in the following manner: Cys(S-4-methylbenzyl), Ser(O-benzyl), Tyr(0-2,6-dichlorobenzyl), and Arg(tosyl). The N-terminal L-pyroglutamine needs no protection at all. Coupling of amino acid residues is achieved by using the BOP (Castro) reagent in the presence of diisopropylethylamine in N,N-dimethylformamide or N-methylpyrrolidone as solvent. All protected amino acids and reagents are in two- to three-fold excess. The HF cleavage is usually conducted in the presence of anisole, thioanisole, and 1,2-ethanedithiol at 0° for 50-60 min. All volatile materials are removed under vacuum and the residue is washed thoroughly with diethyl ether. The peptides are extracted with 15-20% v/v acetic acid, air-oxidized in dilute solution at pH 8 to 8.5 at ambient temperatures, and purified by reversed phase LC on a $C_4$ column.

The preparation of LAS represents a typical synthetic example. All LASA are synthesized using essentially the same procedure, but substituting the desired residues.

The synthesis of LAS starting with 2 g of PMBHA resin hydrochloride (0.35 meq/g) yields 4.147 g of peptidyl resin after completion of the coupling reactions and drying in vacuum. Cleavage of 1 g of the above peptide-resin is achieved by treatment with 30 mL liquid HF, 1.0 mL thioanisole, 0.6 mL ethanedithio, and 0.4 mL anisole at 0° with stirring for 60 min. All volatile materials are removed as rapidly as possible under high vacuum. The residue is washed several times with diethyl ether until practically odorless. It is then extracted with 20% v/v acetic acid and diluted with deionized

water to a volume of 800 mL. The pH of the diluted solution was adjusted to 8.5 by adding concentrated ammonium hydroxide. The resulting solution is stirred at ambient temperature while a stream of air is introduced under the surface for 90 hours. The cloudy solution is mixed with analytical grade Celite and collected on a bed of fresh Celite in a sintered glass funnel. The clear filtrate contains no peptide. The cake of Celite is extracted with 200 mL of 20% v/v acetic acid. The extract is diluted with deionized water and freeze-dried to give 167 mg of the crude oxidized peptide as a white powder. This crude peptide exhibits allatostatin activity in bioassay.

Crude LAS is dissolved in 20% acetic acid and filtered through a 0.02 micrometer membrane filter before purification by reversed phase LC using a Vydac $C_4$ 214TP5415 column and an absorbance detector monitoring at 220 nm. Isocratic elution is carried out with a solvent system of 27% v/v acetonitrile / 0.1% TFA at a flow rate of 1.0 mL/min. Pure LAS is recovered at a retention time of 30 min. The structure is verified by amino acid analysis and MS.

## EXAMPLE 6

Biotechnological Methods: Except as may be otherwise indicated, the following general methods and procedures are employed in carrying out the biotechnological experiments described herein with reference to construction of vectors.

Gene Constructions: DNA fragments are chemically synthesized as are the complementary strands. The strands are annealed to form fragments of the genes and these are ligated into plasmids. The intermediate and final plasmid constructs are analyzed by restriction endonuclease digestion and gel electrophoresis for the presence and positions of diagnostic restriction sites or alternatively by colony blot hybridization using an end labeled oligonucleotide probe corresponding to the inserted gene. The constructs are also sequenced to verify the final sequence.

DNA Synthesis: Single stranded DNA fragments (oligonucleotides) are either commercially available (eg from Clonetech Laboratories, Palo Alto, CA) or synthesized by automated solid phase methods using standard o-methyl or β-cyanoethyl-deoxynucleoside phosphoramidite chemistry (Science 230:281-285, 1985). An Applied Biosystems Model 380A DNA Synthesizer is used for oligonucleotide synthesis using the chemical kits supplied by the manufacturer. Before use in ligation reactions, fragments were phophoylated at the 5' ends, by mixing approximately 3 μg of each oligonucleotide with 5-10 U of $T_4$ polynucleotide kinase in a reaction containing also 20 mM Tris-HCl (pH 7.6), 5 mM $MgCl_2$, 5mM dithiothreitol, 50 μg/ml bovine serum albumin and 1 mM ATP. When used as a hybridization probe, oligonucleotides were labeled at their 5' ends with $^{32}P$ in a similar reaction except that the 1mM ATP was replaced with 10μCi of δ $^{32}P$-ATP (3000 Ci/mmole).

Gene Fragment Construction: Double stranded DNA gene fragments are constructed by mixing approximately 3 μg each of two complementary oligonucleotides in 400 μl of buffered NaCl solution (typically 10 mM Tris-HCl, pH 7.5, 20 mM NaCl, 0.1 mM EDTA); in a 1.5 ml Eppendorf microfuge tube. The mixtures are heated to 90°C for 5 to 20 minutes and slowly cooled to room temperature over a 10 to 60 minute period.

Ligations: Ligations of DNA fragments are performed by standard methods (Maniatis et al. 1982 Molecular Cloning, p. 146) or with the Ligation Kit manufactured by Takara Biochemical Inc. Berkeley, CA according to procedures supplied therewith. Standard ligation reactions are incubated at 4°C for 16 hours. The Takara Kit ligations are incubated at 16°C for 30 minutes. Exact ligation conditions vary depending on the specific reaction. Each ligation is followed by transformation reaction and analysis of the resulting transformants. In general, DNA from selected bacterial colonies isolated after the transformation reaction is prepared using the Mini Scale Preparation described below and analyzed by restriction enzyme digestion and gel electrophoresis. Restriction enzymes appropriate to each specific ligation product are chosen to affirm that the resulting recombinant plasmids are of the intended configuration.

Transformations: Transformation competent MC1061 bacterial cells are prepared by the standard $CaCl_2$ treatment method with the modification that glycerol is added to the cells to 15% and the cells are frozen and stored at -70°C prior to use. To transform cells with recombinant plasmid, between 5 and 20 μl of a ligation reaction is added to 200 μl of freshly thawed competent MC1061 cells and the mixture incubated in an ice bath for 30 minutes. The cells are heat shocked at 42°C for 60 to 90 seconds. Then the culture is plated onto TA or TAXI plates and incubated overnight at 37°C.

Plasmid Mini Scale DNA Preparations: Colonies from transformation plates are picked and inoculated into 1 ml Eppendorf microfuge tubes. The mini cultures are incubated at 37°C for 6 to 18 hours and small scale samples of plasmid DNA are prepared by the rapid boiling method (Maniatis et al., Supra, p. 377), but any of the known available methods may also be used.

| Bacterial Medium: | |
|---|---|
| TUM Medium | 10 g/l Bacto tryptone<br>5 g/l Bacto yeast extract |

(continued)

| Bacterial Medium: | |
|---|---|
| | 5 g/l NaCl<br>0.7 g/l KCl<br>2.5 g/l $MgSO_4.7H_2O$ |
| TUM Plates | TUM Medium plus 15 g/l Bacto agar |
| TA (TUM/Ampicillin) Plates | Tum Plates plus 100 mg/l Ampicillin |
| TAXI Plates | TA Plates plus 50 mg/l Xgal (5-bromo-4-chloro-3- indolyl-β-D-galactoside)<br>40 mg/l IPTG (isopropylthio-β-galactoside) |

Restriction Enzyme Digestions: Restriction endonuclease are purchased from New England Biolabs, International Biotechnologies, Inc., or Promega Biotec and used according to the supplier's specifications. Typically, 20 to 50 µl restriction enzyme reactions are set up using from 1 to 5 µg of plasmid DNA and from 1 to 20 U of enzyme. The reactions also contain 1/10th volume of 10X reaction buffer, either the buffer from the enzyme supplier or one of the three standard restriction enzymes buffers (Mainates et al., Supra, p. 453).

Agarose Gel Electrophoresis: DNA fragments are analyzed on gels containing from 0.5 to 4% agarose (BRL Agarose, BRL LMP Agarose or FMC NuSieve GTG Agarose), 0.5 µg/ml ethidium bromide, 40 mM Tris-Acetate, pH 8.3, 1 mM ethylenediaminetetraacetic acid. The electrophoresis is conducted by standard methods as described by Maniatis et al., Supra, in horizontal electrophoresis tanks. The ethidium bromide stained DNA fragments are visualized using shortwave light and photographed on a UV transilluminator with a Polaroid 4X5 camera and Polaroid Type 57 film. Unknown fragment sizes are determined by comparison with adjacent DNA size standards (BRL 2kb ladder or a Lambda phage HindIII digest). When fragments are purified using gel electrophoresis, the desired fragment is excised from the agarose gel and electroeluted into a small piece of dialysis tubing.

Colony Blot Hybridization: Transformed colonies are transferred to nitrocellulose filters. The colonies are lysed and then the released DNA is affixed to the nitrocellulose using methods described in Sambrook et.al., 1989 Molecular Cloning 2 ed. p. 1.98. The filters were hybridized with $^{32}P$-end labeled oligonucleotide fragments corresponding to the inserted gene(s) using methods described in Sambrook et.al. Supra, pp 1.101-1.104. Hybridized filters were then exposed to x-ray film and plasmid DNA from positive colonies are subjected to DNA sequence analysis to affirm the integrity of the inserted synthetic gene.

DNA Sequencing: Plasmids containing parts of the synthetic gene are sequenced using the Sanger dideoxy method or a variation thereof. Plasmid infected cells are co-infected with the helper phage, M13K07, and single stranded DNA is produced and purified as indicated by the manufacturer, Pharmacia Inc. DNA sequencing reactions are performed using either the Sequenase Kit from US Biochemicals or the Dideoxy Sequencing Kit from Dupont/NEN. Reactions and gels are set up essentially as described in protocols supplied by the kit manufacturers.

## EXAMPLE 7

Production of Recombinant AcMNPV virus: specific methods and procedures

I. Construction of Recombinant AcMNPV.

A. Purification of Viral Particles for Extraction of DNA

Wild type viral DNA for construction of a recombinant virus is prepared from polyhedral inclusion bodies from infected larvae. DNA from non-occluded recombinant viruses for restriction analysis is prepared from infected cells culture fluid.

1. Preparation of virus from cultured cells is as follows. Cell monolayers of $5x10^6$ cells in 150 $cm^2$ flasks are prepared for infection. The medium is removed and viral solution containing $5x10^6$ to $5x10^7$ PFU is added to the flask. After incubation for 60 minutes with rocking by hand at 15 minute intervals, or continual rocking on a mechanical platform, 45 ml of culture medium is added. The medium from the infected cells is collected at 60-72 hours post-infection and centrifuged at 3000 rpm for 20 minutes at 5°C. The supernatant is then brought up to 70 ml with TC-100 and divided between 2 clear ultracentrifuge tubes. Each is underlayered with 3 ml of 25% sucrose in insect saline (1.7 mM PIPES buffer, pH 6.5, 4 mM NaCl, 40 mM KCl, 18 mM $MgCl_2$, 3 mM $CACl_2$), and centrifuged at 25,000 rpm in an SW-28 (Beckman) rotor for 75 minutes to pellet the viral particles.

2. For viral purification from larvae, the polyhedral inclusion bodies are first purified. A large quantity of the viral

particles are purified as follows. Larvae of the Tricoplusia at the first day of the fourth instar are infected orally with virus by application of a suspension containing approximately $10^6$ polyhedra to food in small covered cups. Dead larvae are collected between 3 and 6 days after infection and placed into 25-30 ml of 1% SDS in distilled water. The bodies of the dead larvae are placed into a blender and blended for 30 seconds at high speed and the resulting suspension is filtered through four layers of cheesecloth. The filtered suspension is centrifuged at 6,000 rpm for 10 minutes in Sorvall SS-34 rotor and the pellet is resuspended by sonication in 1% SDS. This centrifugation and sonication is repeated twice, and the suspension is then poured into a conical centrifuge tube and centrifuged for 2 minutes at 400 rpm. The supernatant is collected carefully and centrifuged again at 6,000 rpm for 10 minutes. The clean polyhedra are resuspended in water and their concentration determined by counting a diluted suspension with a hemacytometer.

A suspension containing about $10^{10}$ polyhedra is centrifuged and the pellet is re-suspended in about 15 ml of distilled water with the aid of 20 seconds of sonication. An equal volume of fresh 0.1 M $Na_2CO_3$ is then added. After 15 minutes incubation at room temperature, the solution turns clear and opalescent. It is then centrifuged at 6,000 rpm for 10 minutes and the supernatant is transferred to clear ultracentrifuge tubes and centrifuged again at 18,000 rpm. After centrifugation, the virus pellet is resuspended in 500 μl 10mM Tris, pH7.6, 10 mM EDTA.

B. Preparation of Viral DNA

1. Wild type viral DNA for cotransfection is prepared from polyhedra from infected larvae, purified as described above. Protein removal is important in the preparation of NPV DNA, but the large size of the genome requires that the DNA be handled gently to prevent shearing and degradation.

To the resuspended virus pellet is added 5 μl of 10% SDS solution and 100 μg of Proteinase K for each $10^6$ polyhedra in the starting suspension and the mixture is agitated gently. The sample is then incubated on a shaker at 37°C for 3 to 4 hours, or alternatively placed at 65°C for 1 hour. When the pellet is dissolved, the sample is transferred to a screw-capped tube for extraction. Extractions are done according to the procedure described by Miller, et.al. (1986), p. 294. The purified DNA solution may be either dialysed against 10 mM tris, pH7.6, 1 mM EDTA with at least three changes of dialysis buffer, or may be precipitated in ethanol as described by Miller, et.al. (1986), p. 294.

2. Recombinant viral DNA is prepared from virus recovered from medium of infected cells in culture, as described above. After centrifugation, the supernatant is discarded and the pellet is taken up in 0.5 ml of 10 mM tris, pH7.6, 10 mM EDTA, pH 7.6, 0.25% SDS, 100 μg proteinase K and treated in the same manner as that described for polyhedra-derived viral DNA, above.

C. Cotransfection of Recombinant Plasmid and Wild Type Viral DNA

Recombinant viruses are constructed by homologous recombination between recombinant plasmid DNA and wild type viral DNA in calcium-mediated cotransfected cells. The method for cotransfection is similar to that used in mammalian cells and is described by Miller et.al. (1986), p.295. Polyhedral inclusion bodies can be seen 4 to 5 days after transfection. The cell culture fluid is subjected to a plaque assay for isolation of a recombinant virus.

D. Isolation of a Recombinant Virus Carrying LAS gene

Recombinant virus in which the polyhedrin gene is replaced with the LAS gene is detected by the absence of production of inclusion bodies in infected nuclei using a light microscope. To isolate the recombinant virus from the wild type virus, the plaque assay described herein is used. The cell monolayer infected with a cotransfected virus sample is overlaid by 0.5% SeaKem agarose and cultured for 5 to 8 days. Generally, only between 0.2 to 3% of the produced plaques are occlusion negative.

The culture fluid at 5 to 7 days after cotransfection is collected, centrifuged to remove cells and polyhedral inclusion bodies and diluted for plaque assay. Routinely, the following sets of solutions are prepared by dilution in TC-100 with 10% FCS. (A) $10^{-2}$ dilution; 10 μl of the transfected cell supernatant is mixed into 1 ml of medium; (B) $10^{-4}$ dilution; 10 μl of solution (A) is mixed into 1 ml of medium; (C) $10^{-5}$ dilution; 100 μl of solution (B) is added to 900 μl of medium; (D) $10^{-6}$ dilution; 100 μl of solution (C) is added to 900 μl of medium; (E) $10^{-7}$ dilution; 100 μl of (D) is added to 900 μl of medium; (F) $10^{-8}$ dilution; 100 μl of solution (E) is added to 900 μl of medium. Routinely, only solutions (C), (D), (E) and (F) are assayed. Five hundred of each solution is added to SF-9 cells in a 70 mm dish for plaque assay, as described below.

Plaques produced by the recombinant virus are screened by a light microscope using a combination of low (x20, dissecting scope) and high (x250) magnification. This procedure is described in detail by Miller, et.al. (1986), p.287.

Staining with neutral red on a second overlay is recommended to facilitate the identification of recombinant viruses by light microscopy. When dishes are observed at low magnification, the wild type plaques show more opacity and refractility than recombinant plaques. The candidate recombinant plaques are confirmed at high magnification by the lack of polyhedral inclusion bodies and also by such cytopathological effects as expanded nuclei, lower cell density, or shrunken, shriveled cells.

The plaques produced by a recombinant virus are marked with a felt tipped pen, and are picked with a sterile Pasteur pipet. The Pasteur pipet is stuck vertically into the plaque, and agarose plug is added directly to a well of a 24-well tissue culture dish containing $5x10^5$ cells per well and allowed to incubate for 4 days. In this way, nearly pure recombinanat plaques can be identified and the best ones can be subjected to another round of plaque assay. The plaque purification procedure should be repeated at least twice after the initial assay of the cotransfection mixture to insure a pure clone.

Recombinant AcMNPV virus with LAS gene at the expected site is recovered from the foregoing experiment as confirmed by hybridization and restriction endonuclease analysis.

II. Insect Cell Line

A. Spodoptera frugiperda cells from the SF-9 (freely available from ATCC) cell line are employed and maintained in a culture medium TC-100 which is supplemented with 10% fetal calf serum (FCS). This medium is obtained as a powder from Gibco and is prepared according to package directions. The composition of the medium is listed below. Each liter of medium is supplemented with 100 ml of Fetal Calf Serum (FCS) and 100,000 units penicillinG, 100,000 µg stretomycin and 1,250 µg amphotericin B. Confluent cells are suspended in fresh medium and seeded in fresh flasks at a dilution ratio of 1:5 to 1:7. The cells are generally passaged every 3 to 4 days.

| TC-100 INSECT MEDIUM | |
|---|---|
| Component | mg/ml |
| **INORGANIC SALTS:** | |
| $CaCl_2$ (anhyd.) | 996.49 |
| KCl | 2870.00 |
| $MgCl_2$ (anhyd.) | 1067.95 |
| $MgSO_4$ (anhyd.) | 1357.67 |
| NaCl | 500.00 |
| $NaH_2PO_4•H_2O$ | 1008.36 |
| **OTHER COMPONENTS:** | |
| Glucose | 1100.00 |
| Tryptose | 2000.00 |
| **AMINO ACIDS:** | |
| L-Alanine | 225.00 |
| L-Arginine.HCL | 665.11 |
| L-Aspartic acid | 350.00 |
| L-Asparagine | 350.00 |
| L-Glutamic acid | 600.00 |
| L-Glutamine | 600.00 |
| Glycine | 650.00 |
| L-Histidine.HCl.$H_2O$ | 3697.89 |
| L-Isoleucine | 50.00 |
| L-Leucine | 75.00 |
| L-Lysine.HCl | 625.00 |
| L-Methionine | 50.00 |
| L-Proline | 350.00 |
| L-Phenylalanine | 150.00 |

(continued)

| TC-100 INSECT MEDIUM | |
|---|---|
| Component | mg/ml |
| **AMINO ACIDS:** | |
| L-Serine | 550.00 |
| L-Threonine | 175.00 |
| L-Valine | 100.00 |
| L-Cystine.2HCl | 28.68 |
| L-Tryptophan | 100.00 |
| L-Tryosine.2Na.$2H_2O$ | 72.08 |
| **VITAMINS** | |
| Biotin | 0.01 |
| D-Ca Pantothenate | 0.11 |
| Folic acid | 0.02 |
| i-Inositol | 0.02 |
| Nicotinic acid (Niacin) | 0.02 |
| Para-aminobenzoic acid (PABA) | 0.02 |
| Pyridoxine.HCl | 0.02 |
| Riboflavin | 0.02 |
| Thiamine.HCl | 0.52 |
| Vitamin $B_{12}$ | 0.01 |

B. Preparation of TC-100 culture medium

The medium is obtained as pre-prepared packet of powder from Gibco and is prepared according to the package directions. The powdered medium is dissolved in water, as specified, and 0.35g $NaHCO_3$ and 2.6g Tryptose broth are added per liter. The pH is adjusted to 6.2 with 6N NaOH and the medium is filter sterilized through 2µm. To each liter is added 100 ml of heat inactiviated Fetal Calf Serum (FCS) and 10 ml of a Penicillin-Strepto mycin solution (100,000 units penicillin G, 100,000 µg streptomycin) and 5 ml of Fungizone™ solution (1.250 µg amphotericin B).

III. Plaque Assay

The procedure for plaque assay of AcNPV on SF-9 cells is described by Miller, et.al. (1986), p. 296. A monolayer of $2x10^6$ cells in a 60 mm dish is used for plaque assay. The cells are seeded at least 1 hour prior to plaque assay and the dish should be rocked immediately after the addition of the cells to produce a uniform cell density.

For viral infection, the culture fluid is aspirated from the dish and 500 µl of the viral suspension is added. After incubation for 1 hour with rocking by hand at 15 minute intervals, or continual gentle rocking on a mechanical rocker platform, the inoculum solution is aspirated and the cell monolayers are overlaid with 5 ml TC-100 medium containing 0.5% SeaKem agarose and 10% FCS.

The overlay solution is prepared as follows. SeaKem agarose is suspended at 5% in distilled water in a bottle and autoclaved. The bottle is kept at 42°C after autoclaving. The amount of agarose and distilled water is calculated by the following equations.

$$\text{Agarose (mg)}=(\text{number of plates}+1) \times 25 \text{ mg}$$

$$\text{Water (ml)}=(\text{number of plates}+1) \times 0.5 \text{ ml}$$

Pre-warmed (42°C) TC-100 medium containing 10% FCS is added into the agarose solution and mixed immediately. The amount is calculated as follows:

TC-100(ml)=(number of plates+1) x 4.5 ml

To prevent detachment of the cells, the agarose solution (5 ml) is pipeted slowly onto the infected monolayer. The overlaid plates are kept on the bench to solidify and then transferred to 27°C incubator. Plaques can be identified after incubation for 4 to 8 days. Recombinant plaques may take longer to see clearly than wild type plaques. The titer of the original solution is calculated by dilution rate and the volume added to the plate. To help visualize the plaques, a second overlay (3 ml) containing 0.02% neutral red is recommended. The counting of plaques should be done at least 12 hours after the second overlay.

The viral solution added to the plate for titration should be diluted to give between 10 to 200 plaques per dish. The viral titer in infected medium can be estimated by evaluating the conditions of infection. Generally, the maximum titer is around $10^{10}$ Plaque Forming Units (PFU) per ml in the medium. The lowest titer observed, for example in the medium of the initially infected cells after several washes, is $10^3$ to $10^4$ PFU/ml.

Viral titers can be also estimated as TCID50 using a 96 well plate.

Purified agarose, SeaKem (FMC Corporation) is found to be suited as an overlay material because of a low toxicity to insect cells. The overlay solution contains TC-100, 10% FCS, and 0.5% SeaKem agarose.

IV. Expression of Recombinant Infected Cells in Culture

Expression of LAS in cells infected with the recombinant virus is assayed by immunodetection in Western blot. The recombinant AcNPV is used to infect cells in culture at Multiplicity of Infection (MOI) of 10 and the supernatant and infected cells are harvested, separated by centrifugation and mixed with equal volume of 2xSDS Sample Buffer (125 mM Tris, pH 6.8, 4% SDS, 200 mM Dithiothreitol, 20% glycerol, 0.0016% bromophenol blue), boiled 3 minutes and stored frozen until assay.

The samples are analyzed by separation of components on a polyacrylamide gel, electrophoretic transfer of protein from the gel to nitrocellulose paper, and use of a specific antibody raised agains LAS to detect the presence of the protein in infected cells or supernatant.

<u>Summary of References</u>


Snyder et.al., <u>Cell</u> v.29, 1027-1040, (1982)
USP 4,745,057
Manual of Methods for Baculovirus Vectors & Insect Cell Culture Procedures, Summers & Smith, Texas A&M University, 1988
EP 175852
USP 4,911,913
EP 155476
EP 228036
WO 88/07082
WO 89/01518
NL 8800198
EP 340359
Luchow & Summers, Virology 170, 3139 (1989)
EP 336341
O'Reilly & Miller L.K., Science, v 245, pp 1110-2 (1989)
L.K. Miller, Proc. Vth International Colloquium on Invertebrate Pathology and Microbial Control, Adelaide, p 446 (1990)
EP 359 714 (US Serno 07/245,429)
Pratt & Tobe, Life Sci., v 14:575-586 (1974)
Caruthers, Science, v 230, pp 281-285 (1985)
Maniatis et. al., Molecular Cloning, Cold Spring Harbor Laboratory (1982)
Sambrook et.al., Molecular Cloning 2 ed., Cold Spring Harbor Laboratory (1989)
Miller D. et. al., Genetic Engineering Principles & Methods, v 8, (Ed Setlow, Hollaender) Plenum Press NY (1986)


The above references are incorporated herein by reference to the methods described therein.

**Claims**

**1.** A polypeptide of the formula A

$$AA_1\text{-}AA_2\text{-}AA_3\text{-}AA_4\text{-}AA_5\text{-}AA_6\text{-}Cys\text{-}AA_8\text{-}AA_9\text{-}AA_{10}\text{-}AA_{11}\text{-}AA_{12}\text{-}AA_{13}\text{-}Cys\text{-}AA_{15} \quad (A)$$

wherein each of the AA residues is independently an amino acid selected from Val, Ile, Leu, Met, Phe, Pro, Trp, Asn, Gln, Gly, Ser, Thr, Arg, His, Lys, Asp, Tyr, Ala or Glu or if it is the terminal group each of $AA_1$ to $AA_5$ may be hydrogen; and

wherein the polypeptide is substantially free from associated insect polypeptides and inhibits biosynthesis of juvenile hormone (JH) I, II and III by the corpora allata in Manduca sexta.

**2.** A polypeptide according to Claim 1, wherein each of $AA_6$ to $AA_{15}$ is independently an amino acid selected from Gln, Tyr, Asn, Pro, Ile, Ser, Phe or Ala; and each of $AA_1$ to $AA_5$ is independently an amino acid selected from Glu, Val, Arg, Phe, Ala or Lys or if it is the terminal group, may be hydrogen.

**3.** A polypeptide according to Claim 1, which has the sequence pGlu-Val-Arg-Phe-Arg-Gln-Cys-Tyr-Phe-Asn-Pro-Ile-Ser-Cys-Phe.

**4.** A polypeptide according to Claim 1, wherein $AA_1$ is pGlu, $AA_2$ is Val, $AA_6$ is Gln, $AA_9$ is Phe, $AA_{10}$ is Asn, $AA_{12}$ is Ile, $AA_{13}$ is Ser, $AA_{15}$ is Phe and

a) $AA_3$ and $AA_5$ are both Arg or Lys, $AA_4$ is Phe or Ala, $AA_8$ is Tyr or Phe and $AA_{11}$ is Pro or Ala; or

b) $AA_3$ and $AA_5$ are both Arg or Lys, $AA_4$ is Phe, $AA_8$ is Tyr and $AA_{11}$ is Pro; or

c) $AA_3$ and $AA_5$ are both Arg or Lys, $AA_4$ is Phe or Ala, $AA_8$ is Tyr and $AA_{11}$ is Pro; or

d) $AA_3$ and $AA_5$ are both Arg, $AA_4$ is Phe, $AA_8$ is Tyr or Phe and $AA_{11}$ is Pro; or

e) $AA_3$ and $AA_5$ are both Arg, $AA_4$ is Phe, $AA_6$ is Tyr and $AA_{11}$ is Pro or Ala.

**5.** A polypeptide according to Claim 1, wherein $AA_6$ to $AA_{15}$ has the sequence

**SEQ ID No. 2:** Gln-Cys-Tyr-Phe-Asn-Pro-Ile-Ser-Cys-Phe

and $AA_1$ to $AA_5$ has a sequence selected from pGlu; Arg; Phe-Arg; Arg-Phe-Arg;

**SEQ ID No. 3:** Val-Arg-Phe-Arg;

**SEQ ID No. 4:** pGlu-Val-Arg-Phe-Arg;
Val-Arg-Arg; or

**SEQ ID No. 5:** pGlu-Arg-Phe-Arg.

6. A polypeptide according to any one of Claims 1 to 5, wherein the carboxy terminus is in free form or in amidated form.

7. A recombinant DNA molecular comprising a DNA sequence which encodes a polypeptide according to any one of Claims 1 to 6.

8. A hybrid baculovirus competent to lethally infect insect cells and comprising baculovirus DNA and a DNA sequence coding for a polypeptide according to any one of Claims 1 to 6 , said coding sequence being under expression control of a promoter operable to effect expression of said coding sequence in the baculovirus.

9. A baculovirus according to Claim 8, which is derived Bombyx mori or Autographa californica.

10. An insecticidal composition comprising an insecticidally effective amount of a hybrid baculovirus of Claim 8 and an inert carrier.

11. A method of combatting insects susceptible to a nuclear polyhedrosis baculovirus comprising applying to the locus of the insects an insecticidally effective amount of a hybrid baculovirus of Claim 9 in an inert carrier.

**Patentansprüche**

1. Polypeptid der Formel A

$$AA_1-AA_2-AA_3-AA_4-AA_5-AA_6-Cys-AA_8-AA_9-AA_{10}-AA_{11}-AA_{12}-AA_{13}-Cys-AA_{15} \quad (A)$$

worin jeder der AA-Reste unabhängig voneinander eine Aminosäure, ausgewählt aus Val, Ile, Leu, Met, Phe, Pro, Trp, Asn, Gln, Gly, Ser, Thr, Arg, His, Lys, Asp, Tyr, Ala oder Glu ist oder, sofern die terminale Gruppe, jeder der Reste $AA_1$ bis $AA_5$ Wasserstoff sein kann; und

wobei das Polypeptid im wesentlichen frei von assoziierten Insektenpolypeptiden ist und die Biosynthese von Juvenilhormon (JH) I, II und III durch die Corpora allata in Manduca sexta hemmt.

2. Polypeptid nach Anspruch 1, worin jeder der Reste $AA_6$ bis $AA_{15}$ unabhängig voneinander eine Aminosäure, ausgewählt aus Gln, Tyr, Asn, Pro, Ile, Ser, Phe oder Ala; und jeder der Reste $AA_1$ bis $AA_5$ unabhängig voneinander eine Aminosäure, ausgewählt aus Glu, Val, Arg, Phe, Ala oder Lys ist oder, sofern die terminale Gruppe, Wasserstoff sein kann.

3. Polypeptid nach Anspruch 1, das die Sequenz pGlu-Val-Arg-Phe-Arg-Gln-Cys-Tyr-Phe-Asn-Pro-Ile-Ser-Cys-Phe ist.

4. Polypeptid nach Anspruch 1, worin $AA_1$ pGlu, $AA_2$ Val, $AA_6$ Gln, $AA_9$ Phe, $AA_{10}$ Asn, $AA_{12}$ Ile, $AA_{13}$ Ser, $AA_{15}$ Phe ist und

a) $AA_3$ und $AA_5$ beide Arg oder Lys sind, $AA_4$ Phe oder Ala ist, $AA_8$ Tyr oder Phe ist und $AA_{11}$ Pro oder Ala ist; oder

b) $AA_3$ und $AA_5$ beide Arg oder Lys sind, $AA_4$ Phe ist, $AA_8$ Tyr ist und $AA_{11}$ Pro ist; oder

c) $AA_3$ und $AA_5$ beide Arg oder Lys sind, $AA_4$ Phe oder Ala ist, $AA_8$ Tyr ist und $AA_{11}$ Pro ist; oder

d) $AA_3$ und $AA_5$ beide Arg sind, $AA_4$ Phe ist, $AA_8$ Tyr oder Phe ist und $AA_{11}$ Pro ist; oder

e) $AA_3$ und $AA_5$ beide Arg sind, $AA_4$ Phe ist, $AA_6$ Tyr ist und $AA_{11}$ Pro oder Ala ist.

5. Polypeptid nach Anspruch 1, worin $AA_6$ bis $AA_{15}$ die Sequenz

SEQ ID NO. 2: Gln-Cys-Tyr-Phe-Asn-Pro-Ile-Ser-Cys-Phe

besitzt
und $AA_1$ bis $AA_5$ eine Sequenz, ausgewählt aus pGlu; Arg; Phe-Arg; Arg-Phe-Arg;

SEQ ID NO. 3: Val-Arg-Phe-Arg;

SEQ ID NO. 4: pGlu-Val-Arg-Phe-Arg;
Val-Arg-Arg; oder

SEQ ID NO. 5: pGlu-Arg-Phe-Arg

besitzen.

**6.** Polypeptid nach einem der Ansprüche 1 bis 5, worin das carboxylterminale Ende in freier Form oder in amidierter Form vorliegt.

**7.** Rekombinantes DNA-Molekül, umfassend eine DNA-Sequenz, die ein Polypeptid nach einem der Ansprüche 1 bis 6 codiert.

**8.** Hybrides Baculovirus mit der Kompetenz, Insektenzellen letal zu infizieren, und umfassend Baculovirus-DNA und eine DNA-Sequenz, die ein Polypeptid nach einem der Ansprüche 1 bis 6 codiert, wobei die codierende Sequenz unter Expressionskontrolle eines Promotors ist, der funktionell die Expression der codierenden Sequenz in dem Baculovirus bewirkt.

**9.** Baculovirus nach Anspruch 8, das von Bombyx mori oder Autographa californica abgeleitet ist.

**10.** Insekticide Zusammensetzung, umfassend eine insecticidwirksame Menge eines hybriden Baculovirus nach Anspruch 8 und einen inerten Träger.

**11.** Verfahren zur Bekämpfung von Insekten, die gegenüber einem nucleären Polyhedrosis-Baculovirus empfindlich sind, umfassend die Aufbringung einer insecticidwirksamen Menge eines hybriden Baculovirus nach Anspruch 9 in einem inerten Träger auf den Ort der Insekten.

**Revendications**

**1.** Un polypeptide de formule A

$$AA_1\text{-}AA_2\text{-}AA_3\text{-}AA_4\text{-}AA_5\text{-}AA_6\text{-}Cys\text{-}AA_8\text{-}AA_9\text{-}AA_{10}\text{-}AA_{11}\text{-}AA_{12}\text{-}AA_{13}\text{-}Cys\text{-}AA_{15} \quad (A)$$

sans laquelle chacun des résidus AA est indépendamment un acide aminé choisi parmi Val, ne, Leu, Met, Phe, Pro, Trp, Asn, Gln, Gly, Ser, Thr, Arg, His, Lys, Asp, Tyr, Ala ou Glu ou, s'il est le groupe terminal, chacun des résidus $AA_1$ à $AA_5$ peut être un hydrogène; et
où le polypeptide est sensiblement dépourvu de polypeptides d'insectes associés et inhibe la biosynthèse de l'hormone juvénile (JH) I, II et III par les corps allates de Manduca sexta.

**2.** Un polypeptide selon la revendication 1, dans lequel chacun des résidus $AA_6$ à $AA_{15}$ est indépendamment un

acide aminé choisi parmi Gln, Tyr, Asn, Pro, Ile, Ser, Phe ou Ala; et chacun des résidus $AA_1$ à $AA_5$ est un acide aminé choisi parmi Glu, Val, Arg, Phe, Ala ou Lys ou, s'il s'agit du groupe terminal, peut être un hydrogène.

**3.** Un polypeptide selon la revendication 1, qui a la séquence pGlu-Val-Arg-Phe-Arg-Gln-Cys-Tyr-Phe-Asn-Pro-Ile-Ser-Cys-Phe.

**4.** Un polypeptide selon la revendication 1, dans lequel $AA_1$ est pGlu, $AA_2$ est Val, $AA_6$ est Gln, $AA_9$ est Phe, $AA_{10}$ est Asn, $AA_{12}$ est Ile, $AA_{13}$ est Ser, $AA_{15}$ est Phe et

a) $AA_3$ et $AA_5$ sont tous deux Arg ou Lys, $AA_4$ est Phe ou Ala, $AA_8$ est Tyr ou Phe et $AA_{11}$ est Pro ou Ala; ou
b) $AA_3$ et $AA_5$ sont tous deux Arg ou Lys, $AA_4$ est Phe, $AA_8$ est Tyr et $AA_{11}$ est Pro ; ou
c) $AA_3$ et $AA_5$ sont tous deux Arg ou Lys, $AA_4$ est Phe ou Ala, $AA_8$ est Tyr et $AA_{11}$ est Pro ; ou
d) $AA_3$ et $AA_5$ sont tous deux Arg, $AA_4$ est Phe, $AA_8$ est Tyr ou Phe et $AA_{11}$ est Pro ; ou
e) $AA_3$ et $AA_5$ sont tous deux Arg, $AA_4$ est Phe, $AA_6$ est Tyr et $AA_{11}$ est Pro ou Ala.

**5.** Un polypeptide selon la revendication 1, dans lequel $AA_6$ à $AA_{15}$ a la séquence

SEQ ID N°2: Gln-Cys-Tyr-Phe-Asn-Pro-Ile-Ser-Cys-Phe

et $AA_1$ à $AA_5$ a une séquence choisie parmi pGlu ; Arg; Phe-Arg, Arg-Phe-Arg ;

SEQ ID N° 3 : Val-Arg-Phe-Arg ;

SEQ ID N° 4 : pGlu-Val-Arg-Phe-Arg ;
Val-Arg-Arg ; ou

SEQ ID N° 5 : pGlu-Arg-Phe-Arg.

**6.** Un polypeptide selon l'une quelconque des revendications 1 à 5, dans lequel l'extrémité carboxy est sous forme libre ou sous forme amidée.

**7.** Un ADN recombinant moléculaire comprenant une séquence d'ADN qui code pour un polypeptide selon l'une quelconque des revendications 1 à 6.

**8.** Un baculovirus hybride compétent pour infecter léthalement des cellules d'insectes et comprenant de l'ADN de baculovirus et une séquence d'ADN codant pour un polypeptide selon l'une quelconque des revendications 1 à 6, ladite séquence codante étant sous le contrôle d'expression d'un promoteur permettant d'effectuer l'expression de ladite séquence codante dans le baculovirus.

**9.** Un baculovirus selon la revendication 8, qui est dérivé de Bombyx mori ou d'Autographa californica.

**10.** Une composition insecticide comprenant une quantité efficace comme insecticide d'un baculovirus hybride de la revendication 8 et un véhicule inerte.

**11.** Un procédé pour combattre les insectes sensibles au baculovirus de la polyédrose nucléaire, comprenant l'application au locus des insectes d'une quantité efficace comme insecticide d'un baculovirus hybride de la revendication 9 dans un véhicule inerte.